(19) Europäisches Patentamt
European Patent Office
Office européen des brevets

(11) Publication number: **0 292 176 A2**

(12) # EUROPEAN PATENT APPLICATION

(21) Application number: 88304205.3

(22) Date of filing: 09.05.88

(51) Int. Cl.$^4$: C07F 15/04 , C07C 149/34 , C07D 339/06 , C08K 5/00

Claims for the following Contracting State: ES.

(30) Priority: 21.05.87 GB 8712033
21.05.87 GB 8712034
29.10.87 GB 8725325

(43) Date of publication of application: 23.11.88 Bulletin 88/47

(84) Designated Contracting States: AT BE CH DE ES FR GB GR IT LI LU NL SE

(71) Applicant: IMPERIAL CHEMICAL INDUSTRIES PLC
Imperial Chemical House Millbank
London SW1P 3JF(GB)

(72) Inventor: Stark, William Marshall
Institute of Genetics Glasgow University
Church Street, Glasgow G11 5JS
Scotland(GB)

(74) Representative: James, David Gomer et al
Imperial Chemical Industries PLC Legal Department: Patents Po Box 6 Bessemer Road
Welwyn Garden City Herts, AL7 1HD(GB)

(54) **Organo-metal compositions, preparation and use.**

(57) Complexes of a transition metal and a tetrasubstituted aromatic compound which contain at least two transition metal atoms. At least two of the substituents are -SH groups, the other two substituents being -SH, -OH and -NHR. The complexes are prepared by reacting the tetrasubstituted aromatic compound with a transition metal compound in the presence of a disubstituted aromatic compound. Depending on the relative proportions of the di- and tetra-substituted compounds, the resulting complex may contain more than one residue from the tetra-substituted compound and more than two transition metal atoms. The complexes possess infra-red absorbing properties which vary in dependence on the particular complex, the complexes with more than one residue from the tetrasubstituted compound absorbing radiation of longer wavelength. The compounds can be used to provide infra-red absorbing compositions, for example as a coating or inter-layer for glass. The complexes may be used together with one or more different infra-red absorbing materials. The tetra-substituted aromatic compound may be a tetra-thiol and this can be prepared from an aromatic compound containing at least four halogen substituents by reaction with an alkaline thiol compound in the presence of iron and sulphur in a polar solvent such as dimethyl formamide.

EP 0 292 176 A2

## ORGANO-METAL COMPOSITIONS, PREPARATION AND USE

The present invention relates to organo-metal compositions, the preparation of such compositions and of precursors thereof and the use of such organo-metal compositions as a component of an infra-red absorbing material.

Metal complexes of benzene-1,2-dithiols have been described and are disclosed as having absorption characteristics in the infra-red zone of the spectrum. Compounds having infra-red absorbing characteristics are of importance in many fields. In many applications the compounds of this type can be incorporated into plastics materials which are then formed into film or sheet which can be used, for example, in sun glasses, welder's goggles or absorbing coatings on windows or in the inter-layer between sheets of glass in laminated glass. Plastic film containing an infra-red absorber can also be used for agricultural purposes and increasing use in being made of polyethylene or polyvinylchloride for greenhouses.

Metal complexes which may be used to provide infra-red absorbing characteristics are disclosed, inter-alia, in published UK Patent Application No.2116543. Other metal complexes having infra-red absorbing characteristics are disclosed in United States Patent 3850502. The absorption spectra of these complexes differ depending on the particular complex but the spectra are generally similar and absorb only in a part of the total infra-red spectrum. For some applications, for example in plastic film for a greenhouse or a cloche, it is desirable to provide absorption of radiation over a wide range of wavelengths, for example wavelengths from 700nm to 2500nm.

According to the present invention there is provided an organo-metal complex which is at least one compound of the formula:

$$[RYZ\ (MXYArXY)_xMYZR]A$$

wherein:

A is a cation having a valency, and being in an amount, to give a neutral compound;
Ar is an aromatic hydrocarbon or heterocyclic residue having at least four substituents;
R is a hydrocarbon residue having a least two substituents;
M is a transition metal;
X is a sulphur atom or a group $NR^1$;
Y is a sulphur atom, an oxygen atom or a group $NR^1$;
Z is a sulphur atom, an oxygen atom or a group $NR^1$;
$R^1$ is a hydrogen atom or a hydrocarbyl group; and
x is an integer having a value of from 1 up to 10,
where each X, Y and Z may be the same or different.

For convenience hereafter a compound of the formula:

$$[RYZ(MXYArXY)_xMYZR]A$$

will be referred to simply as an "organo-metallic compound".

The cation A is conveniently a monovalent cation. When A is a monovalent cation, each molecule of the organo-metallic compound typically contains (x+1) molecules of the cation A. The cation A may be an ammonium group, which term includes a quaternary ammonium group, an alkali metal such as sodium or potassium, or a cation of higher valency such as, for example, an alkaline earth metal.

The group Ar is an aromatic hydrocarbon or heterocyclic residue containing, as substituents, the groups X and Y. The group Ar may contain further substituents which may be the same as, or different from, the substituents from which X and/or Y are derived. Depending on the preparative technique used to obtain the compound from which the group XYArXY has been obtained, the substituents may, or may not, be electron donating. Thus, the substituents may be groups which are not strongly electron donating and may be for example halogen, alkyl, acyl, aryl, $SR^2$ or CN where $R^2$ is an alkyl group, preferably containing 1 to 30 carbon atoms. However, the presence of substituents which are strongly electron donating is possible, such groups including acyloxy, haloalkyl, acyl halide and sulphonyl halide groups. As is described in more detail hereafter, the group Ar is typically derived from a tetrathiol which can be obtained from a tetrahalo-compound. Haloalkyl, acyl halide and sulphonyl halide groups should not be present in the process for obtaining the tetrathiol from the tetra-halo compound. The group Ar may be the residue of a monocyclic aromatic hydrocarbon such as benzene or of a multi-ring compound, which term is used herein to include two or more rings, such as naphthalene. Alternatively, the group Ar may be the residue of a heterocyclic

compound having aromatic characteristics, such as, for example, pyridine.

In the group Ar, each group X is attached in a position ortho- to a group Y. In a monocyclic system which contains only two of groups X and only two of groups Y, the substituent groups X, Y, X, Y are preferably attached in the 1, 2, 4, 5 positions. The 3 and 6 positions may be occupied by hydrogen atoms or by substituents such as halogen atoms.

The group Ar is conveniently derived from a benzene-1,2,4,5-tetrathiol compound. As is discussed in more detail hereafter, such a compound is conveniently obtained from a halobenzene containing at least four halogen atoms such as, for example, 1,2,4,5-tetrachlorobenzene or hexachlorobenzene.

It is preferred that each group X is a sulphur atom. It is especially preferred that the groups X and Y are all the same.

The value of x is preferably from 1 up to 5 and especially is 1, 2 or 3. A mixture of organo-metallic compounds, wherein the compounds differ only in respect of the value of x is a preferred aspect of the present invention.

M is a transition metal by which is meant a metal of Group IVA, VA, VIA, VIIA, VIII and IB of the Periodic Table. All references herein to the Periodic Table are to the Long Periodic Table as set out on the rear inside cover of "General and Inorganic Chemistry" by J.R.Partington, Second Edition published by MacMillan and Co.Limited, 1954. Many transition metals form coloured materials but we prefer compounds which are essentially uncoloured. Thus we prefer that the metal M is a metal of Group VIII of the Periodic Table. It is especially preferred that M is selected from nickel, palladium or platinum. Useful materials have been obtained when M is nickel.

The group R is typically of the type:

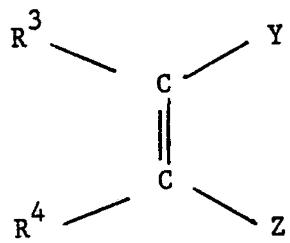

where:

Y and Z are as hereinbefore defined;

$R^3$ and $R^4$, which may be the same or different, are hydrogen, hydrocarbyl or substituted hydrocarbyl groups or heterocyclic groups or $R^3$ and $R^4$, together with the two carbon atoms to which they are attached, may form a ring system.

Preferably at least one of the groups Y and Z is a sulphur atom. It is especially preferred that the groups Y and Z attached to the group R are both the same. Most preferred are those compounds in which each pair of groups X and Y attached to the group Ar are the same as the pair of groups Y and Z attached to the group R.

The groups $R^3$ and $R^4$ may each be an aromatic group. Alternatively, the groups $R^3$ and $R^4$, with the two carbon atoms, may form a ring system which may be substituted. Thus, the group R may be, for example,

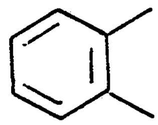

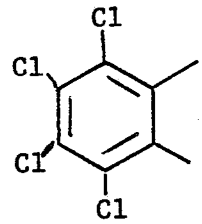

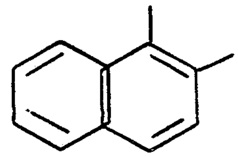

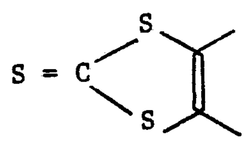

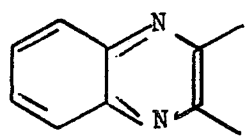

or

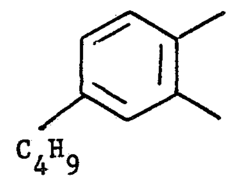

We generally prefer that the group R contains a conjugated double bond system, as in an aromatic ring system.

As is discussed in more detail hereafter, the organo-metallic compound of the present invention is conveniently prepared as an admixture with other organo-metallic compounds in accordance with the invention and such a mixture may also include a compound of the general formula:

[RYZMYZR]A

where A, M, R, Y and Z are all as hereinbefore defined.

Thus, as a further aspect of the present invention there is provided a mixture of compounds of the formula:

$[RYZ(MXYArXY)_xMYZR]A$

wherein A, Ar, M, R, X, Y, Z and x are all as hereinbefore defined x is different in the different compounds.

The mixture may additionally include a compound of the formula:

{RYZMYZR]A

where A, M, R, Y and Z are all as hereinbefore defined. Compounds of this general type are disclosed, inter alia, in UK Patent Application No.2116543 and United States Patent 3850502 and correspond to the compounds of the present invention with the exception that the value of x is zero.

A specific compound in accordance with the present invention is one having the formula:

$[RS_2NiS_2ArS_2NiS_2R]\ 2Bu_4N$

where
$S_2ArS_2$ is derived from 3,6-dichlorobenzene-1,2,4,5-tetrathiol;
$RS_2$ and $S_2R$ is derived from 3,4,5,6-tetrachlorobenzene-1,2-dithiol;
and
Bu is n-butyl.

A further compound in accordance with the present invention is one having the formula:

$[RS_2(NiS_2ArS_2)_2NiS_2R]\ 3Bu_4N$

where
$S_2ArS_2$ is derived from 3,6-dichlorobenzene-1,2,4,5-tetrathiol;
$RS_2$ and $S_2R$ is derived from 3,4,5,6-tetrachlorobenzene-1,2-dithiol;

and
Bu is n-butyl.

The foregoing nickel thiol compounds may be mixed together and also with the higher homologues thereof of the general formula:

$[RS_2(NiS_2ArS_2)_nNiS_2R]$ (n + 1) $Bu_4N$

where R, Ar and Bu are as defined for the foregoing nickel compounds; and n is at least 3 and not more than 10.

The mixture may also include a compound of the formula:

$[RS_2NiS_2R]$ $Bu_4N$

where
$RS_2$ and $S_2R$ is derived from 3,4,5,6-tetrachlorobenzene-1,2-dithiol;
and
Bu is n-butyl.

Compounds in accordance with the present invention can be obtained by the reaction of a salt of the metal M with precursors of the linking groups and the end groups. The reaction may optionally, but not necessarily, be effected in the presence of a base. The reaction is preferably effected in the presence of a solvent for the reactants and the reaction product if the latter is soluble. The reaction mixture is preferably oxidised to obtain the preferred product.

More specifically, according to a further aspect of the present invention there is provided a process for the production of organo-metallic compounds which comprises reacting a salt of a metal (M) with a compound of the formula:

$(HY)_2Ar(XH)_2$

and a compound of the formula:

HYRZH

optionally in the presence of a base;
where Ar, M, R, X, Y and Z are all as hereinbefore defined.

The compound of the formula:

$(HY)_2Ar(XH)_2$

may be, inter alia,
benzene-1,2,4,5-tetrathiol;
3,6-dichloro-1,2,4,5-tetrathiol;
naphthalene-1,2,5,6-tetrathiol;
pyridine-2,3,5,6-tetrathiol;
1,4-dihydroxybenzene-2,5-dithiol;
1,2,4,5-tetraaminobenzene;
1,3-diaminobenzene-4,6-dithiol;
1,4-diaminobenzene-2,5-dithiol;
2,6-dihydroxynaphthalene-1,5-dithiol.

The compound of the formula HYRZH may be, inter alia,
3,4,5,6-tetrachlorobenzene-1,2-dithiol;
naphthalene-1,2-dithiol;
2-hydroxynaphthalene-1-thiol;
2-aminothiophenol;
4-butyl-1,2-diaminobenzene; or
quinoxaline-2,3-dithiol.
Alternatively, compounds of the type:

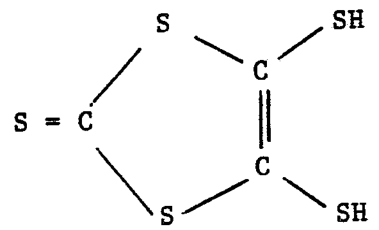

(1,3-dithiole-2-thione-4,5-dithiol) or

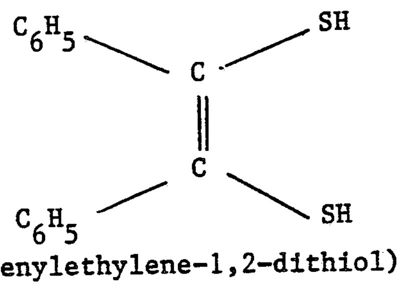

(1,2-diphenylethylene-1,2-dithiol)

may be used but, since these compounds are not stable, they have to be formed, in situ, in the reaction mixture used to produce the organo-metallic compound, but when these compounds are to be generated in situ, the dithiolate anions may be formed, and react, rather than the dithiols.

The salt of the metal (M) may be any suitable salt. Suitable salts include halides, nitrates, and sulphates but other salts may be used. In order to obtain ready reaction of the salt, it is preferred to use a salt which is soluble in the reaction medium. If the metal (M) is nickel, a convenient salt is nickel chloride, which can be used as the hydrated salt.

If a base is used, this can be any suitable base including the oxide, hydride, hydroxide, alkoxide or phenoxide of an alkali metal or an alkaline earth metal. However, the resulting product contains the alkali or alkaline earth metal as the cation (A) and such products are generally of low solubility in both organic and inorganic media. It is generally preferred that the resulting product is soluble to some extent in the reaction medium and hence it is preferred that the cation is of type $(R^1)_4Q^+$. This cation can be present in the base, for example if $(R^1)_4NOH$ is used or added as a salt $(R^1)_4QX^1$ to the reaction where:

$R^1$ is as hereinbefore defined and each $R^1$ may be the same or different;

Q is a non-metallic element selected from nitrogen, phosphorus and arsenic; and

$X^1$ is a monovalent anion.

$R^1$ is preferably an alkyl group, especially one containing from 2 to 24 carbon atoms, especially up to 18 carbon atoms, most especially up to 8 carbon atoms, for example 4 carbon atoms. Q is preferably a nitrogen atom. $X^1$ is conveniently a halogen atom, such as chlorine, bromine or iodine.

Alternativley the reaction may be effected in the presence of an alkali metal hydroxide to form an alkali metal salt which is then reacted with a further compound, for example a compound of the type $(R^1)_4QX^1$ to give the desired product. Whilst the compound $(R^1)_4QX^1$ is conveniently one in which each $R^1$ is the same, for example as in tetrabutyl ammonium bromide, it will be appreciated that the groups $R^1$ may be different, for example as in cetyl trimethyl ammonium bromide.

The reaction is preferably effected in the presence of a liquid in which one or both of the compounds $(HY)_2Ar(XH)_2$ and HYRZX are soluble, in which liquid it is preferred that the salt of metal (M) is also soluble. Suitable liquids are organic liquids, particularly alkanols; glycols; polar aprotic solvents such as N,N-dimethylformamide, dimethylsulphoxide, N-methylpyrrolidone and tetrahydrofuran; and aqueous mixtures such as aqueous alkanols of acetone/water mixtures. However, it will be appreciated that the liquid used will depend on the compounds being reacted and hence the choice of the liquid reaction medium can be varied in accordance with the particular materials being reacted.

The reaction temperature is also dependent on the particular materials being reacted, and also upon the liquid reaction medium. However, in general a temperature of not more than 100°C can be used, in particular a temperature in the range from 0°C to 50°C, for example ambient temperature.

The reaction product is typically a mixture of organo-metallic compounds of the type hereinbefore defined, and may also include a compound of the type [RYZMYZR]A. Hence, the process of the present

invention provides a convenient procedure for obtaining a mixture of compounds in accordance with a further aspect of the present invention. If desired, the compound [RYZMYZR]A, and the organo-metallic compounds in which the value of x is one, two or three can be separated from the reaction product mixture. The compounds containing only a single metal atom, that is the compounds of the formula [RYZMYZR]A are generally more soluble than the other components of the mixture and can be removed from the solid reaction product by washing the solid with a solvent such as chloroform. Compounds of the type in which X is greater than one, and particularly where X is at least two, are less soluble than the compound with x = 1 and can be removed by dissolving the solid reaction product in a solvent such as N,N-dimethylformamide or tetrahydrofuran and filtering to remove the insoluble solid in which the value of x is greater than one.

We have found that if two organo-metallic compounds are mixed together and heated at an elevated temperature, for example at least 50°C and preferably at least 100°C, reaction can occur to form an intermediate product. Thus, if a compound of the type [RYZMYZR]A and a compound of the type [RYZ-(MXYArXY)$_x$MYZR]A, where x has a value If at least two, are mixed together in the presence of a suitable solvent, for example N,N-dimethylformamide, and the mixture is heated, for example under reflux, reaction occurs to give a compound of the type [RYZ(MXYArXY)$_{x'}$MYZR]A, where the value of x$'$ is less than the value of x. Alternatively, two compounds of the type [RYZ(MXYArXY)$_x$MYZR]A, where at least some of the groups R, Y, Z, X, and Ar, and optionally M, A and the value of x, are different in the two compounds, may be treated in a similar manner to obtain an intermediate product having properties, particularly infra-red absorbing characteristics, which are different from those of either of the starting materials.

As is discussed in more detail hereafter, some organo-metallic compounds in accordance with the present invention have infra-red absorbing characteristics. To obtain such products, a further step may be necessary in the process, in particular the initially formed reaction product mixture is subjected to an oxidation step. We have found that mild oxidation conditions are generally satisfactory, for example by bubbling air through the reaction product mixture for a period of time of from 1 minute up to 25 hours, particularly from 1 to 10 hours, for example 3 hours.

Thus, a preferred process in accordance with the present invention comprises reacting a salt of a metal (M) with a compound of the formula $(HY)_2Ar(XH)_2$ and a compound of the formula HYRZH in the presence of a base and subjecting the initial reaction product mixture to an oxidation step. Optionally, the oxidised mixture is then subjected to a separation step to isolate at least some of the products. The nature of the reaction product is dependent on the reactants used and also on the proportions of the reactants. Thus, the reaction mixture may consist of two moles of the salt of the metal (M), one mole of the compound $(HHY)_2Ar(XH)_2$ and two moles of the compound HYRZH to produce a product which is predominantly, or even solely, of the formula:

[RYZ(MXYArXY)MYZR]A

In general such a reaction mixture results in a product mixture which includes a compound of the type [RYZMYZR]A and higher oligomers of the organo-metallic compound in which the value of x is at least two.

If the reaction mixture contains three moles of the salt of the metal (M), two moles of the compound $(HY)_2Ar(XH)_2$ and two moles of the compound HYRZH, the product obtained will contain a substantial proportion of the organo-metallic compound in which the value of x is two.

In general we prefer to use reaction mixtures in which the proportions of the reactants are as previously disclosed or in which the proportions are intermediate between those disclosed. However, it will be appreciated that the use of higher proportions of the salt of the metal (M) and the compound $(HY)_2Ar(XH)_2$ will produce mixtures in which te average value of x is higher, whilst the use of a higher proportion of the compound HYRZH will produce mixtures containing increasing proportions of the compound [RYZMYZR]A and in which the average value of x is reduced.

The proportion of the reactants can be varied to obtain a desired reaction product mixture. Alternatively, two reaction product mixtures of different composition can be prepared and these can be mixed in any desired ratio to obtain an intermediate mixture having a desired composition. Preferred mixtures contain approximately equimolar proportions of the compounds [RYZMYZR]A; [RYZ(MXYArXY)MYZR]A and [RYZ-(MXYArXY)$_2$MYZR]A, together with a further minor proportion of the higher oligomers of the formula [RYZ-(MXYArXY)$_x$MYZR]A, in which the value of x is from 3 up to 10, the mixture containing decreasing proportions of compounds having the higher values of x. In such a mixture M is preferably nickel; X, Y and Z are all sulphur atoms; R and Ar are, optionally substituted, mono-cyclic aromatic rings, and A is a quaternary ammonium cation in a sufficient quantity to balance the charge on the metal complex anion.

In the process of the present invention, a compound which forms a tetradentate ligand is used, namely the compound $(HY)_2Ar(XH)_2$. Many compounds of this type are known and these compounds can be

prepared using processes described in the art, for example in Monatsch, 50, 259; US Patent 4225700; J.Org.Chem. 46 (1981) 3070 and J.Org.Chem. 50 (1985) 2395. Other compounds of this type can be prepared by the reaction of dihydroxyaromatic compounds with sulphur-containing reagents using procedures analogous to those described in GB 383284 for the preparation of the corresponding mono-hydroxy compounds. Thus, for example, 2,6-dihydroxynaphthalene is reacted with sodium thiocyanate in the molecular ratio of 1:2 in the presence of bromine and the thiocyanate derivative obtained is then treated with a mixture of sodium hydroxide and sodium sulphide to produce 2,6-dihydroxynaphthalene-1,5-dithiol.

Preferred compounds for use in the process of the present invention are tetrathiol compounds. We have found a convenient method for the production of such compounds.

Thus, in accordance with a further feature of the present invention, there is provided a process for the production of an aromatic tetrathiol compound wherein an aromatic compound containing at least 4 halogen substituents attached to an aromatic ring is reacted with a metal hydrosulphide and sulphur in the presence of iron powder or an iron salt in a polar organic solvent where for each mole of the aromatic compound there are used at least 4 moles of the hydrosulphide, at least 0.5 moles, preferably about one mole, of sulphur and at least 0.5 moles, preferably about one mole, of iron powder or an iron salt.

The aromatic compound can be tetrachlorobenzene or hexachlorobenzene, but it will be appreciated that other aromatic compounds may be used. The aromatic compound may contain other substituents but the presence of substituents which react with a thiol group is undesirable and such groups should be avoided. Substituents which may be present, other than hydrogen, are halogen atoms, or alkyl, acyl, aryl, alkylthio, alkyloxy and cyano groups, whilst substituents which should be avoided are activated ester and thioester, haloalkyl, acyl halide and acyl anhydride groups.

The hydrosulphide can be the hydrosulphide of an alkali metal or of an alkali earth metal, in particular sodium or potassium hydrosulphide.

It is generally convenient to use iron powder but iron salts such as ferrous chloride can be used if desired.

Suitable polar solvents include alcohols, glycols, amide derivatives such as dimethylformamide, dimethylacetamide, hexamethylphosphoramide and tetramethylurea or sulphur derivatives such as dimethylsulphoxide. We have obtained satisfactory results using dimethylformamide as the polar solvent.

The reaction can be effected at ambient temperature but generally it is preferred to use an elevated temperature which is at least 80°C and preferably at least 100°C. The temperature typically does not exceed 150°C and conveniently is effected at the reflux temperature of the polar solvent, for example about 140°C when the solvent is dimethylformamide.

The reaction product is an iron complex and this is separated from the reaction mixture and then treated with zinc oxide and a base to decompose the complex and liberate the desired tetrathiol compound.

Using 1,2,4,5-tetrachlorobenzene in the foregoing process, we have obtained a good yield of benzene-1,2,4,5-tetrathiol. Using hexachlorobenzene, the product obtained is predominantly 3,6-dichloro-1,2,4,5-tetrathiol.

In a variation of the foregoing process, the proportions of the hydrosulphide, sulphur and iron or iron compound can be reduced when a mixture of a di- and tetra-thiol compound is obtained. According to this variation, more than two moles of the hydrosulphide are used, together with more than 0.5 mole of iron or iron compound. The product mixture of di- and tetra-thiol compounds may then be used in the process for the preparation of the organo-metallic compound or a mixture containing at least one organo-metallic compound.

Compounds of the general type [RYZMYZR]A are known, for example from British Patent Specification 2116543. These compounds have an absorption spectrum in the near infra-red section of the spectrum and have been proposed for use as infra-red absorbers in a number of different applications. However, these compounds absorb in only a limited portion of the infra-red spectrum and it is desirable to provide a material which absorbs radiation over a wide range of wavelengths.

The organo-metallic compounds of the present invention also absorb in the infra-red section of the spectrum but absorb at a longer wavelength than compounds of the general type [RYZMYZR]A. The wavelength at which absorption occurs is dependent on the nature of the organo-metallic compound and particularly on the value of x. Thus, a compound in which the value of x is one, can absorb at a wavelength of about 1200nm whilst in a similar compound in which the value of x is two, absorption typically occurs at a wavelength of about 1400-1500nm. Compounds in which the value of x is greater than two absorb at higher wavelengths, up to a maximum of about 2000nm, as the value of x increases, but no benefit in terms of absorption at higher wavelengths is apparent at values of x in excess of 10. The wavelength at which absorption occurs is dependent both on the structure of the organo-metallic compound and, for a given structure type, depends on the value of x.

Organo-metallic compounds in accordance with the present invention can be used to provide absorption of infra-red radiation in selected portions of the spectrum. However, absorption at a wide range of wavelengths can be achieved by using a mixture of the organo-metallic compounds of the present invention. Absorption over a wider range of wavelengths can be achieved using a mixture which also includes a compound of the general type [RYZMYZR]A. Such a mixture gives absorption at wavelengths of from 850 to 2000nm.

The organo-metallic compounds of the present invention may be incorporated into a range of materials such as glass or plastics material to provide said material with infra-red absorbing properties. The organo-metallic compounds may, in particular, be incorporated into a plastics material, to provide a product having absorbing properties in a limited part of the infra-red spectrum or, by the use of a mixture of the compounds, preferably together with a compound of the general type [RYZMYZR]A, from the near infra-red up to a wavelength of about 2000nm.

The organo-metallic compounds of the present invention, or mixtures thereof, may be used together with other materials, in particular with other materials which have infra-red absorbing characteristics. A wide range of materials are known to have infra-red absorbing characteristics including not only various compounds of the general type

[RYZMYZR]A

but also phthalocyanine, including naphthalocyanine, derivatives; metal-aromatic amine complexes; metal dithiolethionedithiol complexes; aminium or diimonium salts; and other compounds. The organo-metallic compounds of the present invention, or mixtures thereof, may be formed into a composition containing at least one compound having infra-red absorbing characteristics.

Thus, as a further feature of the present invention there is provided a composition comprising at least one compound of the formula

$[RYZ(MXYArXY)_xMYZR]A$

optionally at least one compound of the formula

[RYZMYZR]A

and at least one infra-red absorbing phthalocyanine derivative.

Compounds of the formula

[RYZMYZR]A

which may be present in the composition are particularly a metal-aromatic dithiol complex (hereafter simply a "metal dithiolene") or a metal oxythio complex.

Metal dithiolenes are generally known and are disclosed, inter alia, in published UK Patent Application No.2116543.

The metal dithiolene is typically of the general formula:

$[Ar^1S_2 - M - S_2Ar^1] - A$

wherein

A and M are both as hereinbefore defined; and

$Ar^1$ is a monocyclic aromatic hydrocarbon residue which is derived from an aromatic ortho-dithiol and which may be further substituted.

Preferably the metal M is as preferred in the organo-metallic compound of the present invention.

The cation A is conveniently a monovalent cation and each molecule of the metal dithiolene contains one molecule of the cation A. The cation A may be an ammonium group, which term includes a quaternary ammonium group, or may be an alkali metal such as sodium or potassium or a quaternary phosphonium or arsonium group.

The group Ar is typically of the type

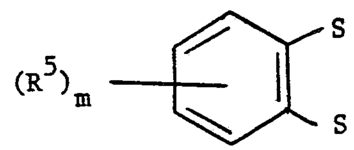

where

each $R^5$ which may be the same or different, is a halogen atom, a hydrocarbyl or substituted hydrocarbyl group, a hydrocarbyloxy group, a hydrocarbylthio group, an amino group, an acyl group, a nitro group or a heterocyclic group; and

m is zero or an integer of one to four.

If the value of m is zero then, except for the sulphur atoms, the group $Ar^1$ is an unsubstituted benzene ring. The groups $R^5$ are preferably halogen atoms, alkyl groups containing from 1 to 24 carbon atoms, particularly from 1 to 6 carbon atoms or acyl groups containing from 2 to 24 carbon atoms, particularly from 2 to 6 carbon atoms as in acetyl, propanoyl and butanoyl. Thus, the group $Ar^1$ may be, for example

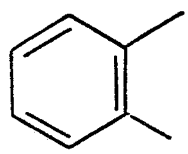

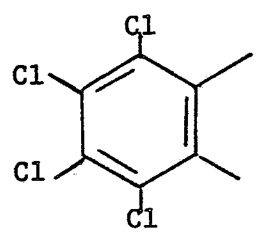

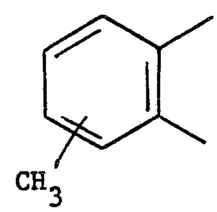

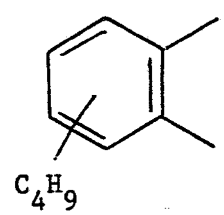

or

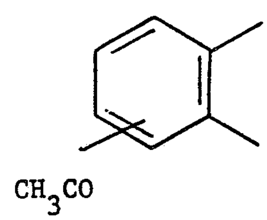

Metal oxythio complexes are generally known and complexes of this type are disclosed, inter alia, in United States Patent No.3850502.

The metal oxythio complex is typically of the general formula

$[Ar^2SOMOSAr^2]A$

where

A and M are both as hereinbefore defined; and

$Ar^2$ is an aromatic hydrocarbon residue which is derived from an aromatic ortho hydroxy-thiol and which may be further substituted.

Preferably the metal M and the cation A are as preferred in the organo-metallic compound of the present invention.

The group $Ar^2$ may contain only one aromatic ring but preferably contains at least two aromatic rings fused together, In general $Ar^2$ contains not more than three rings fused together and especially contains only two rings fused together. The oxygen and sulphur atoms are attached to adjacent carbon atoms in one ring of the group $Ar^2$. The group $Ar^2$ may be substituted by groups additional to the sulphur and oxygen atoms and, if $Ar^2$ is a fused ring system, any such additional substituents may be attached to the ring to which the sulphur and oxygen atoms are attached but it is generally preferred that any such additional substituents are attached to a ring other than that to which the sulphur and oxygen atoms are attached. Thus the group $Ar^2$ is particularly of the type

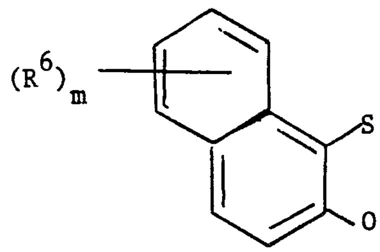

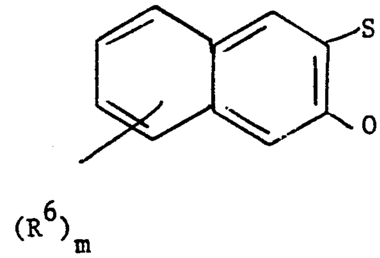

or

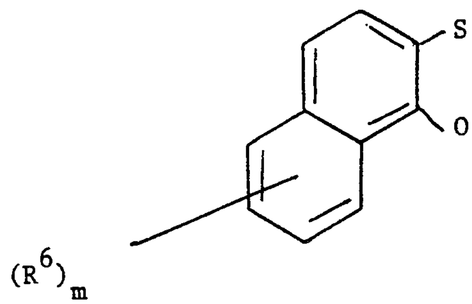

where

m is as hereinbefore defined; and

each $R^6$, which may be the same or different, is a halogen atom, a hydrocarbyl or substituted hydrocarbyl group, an acyl group, a heterocyclic group or a sulphonic acid group, or two of the groups $R^6$, together with the two carbon atoms to which they are attached, form a ring system.

Although two of the groups $R^6$ may, together with the carbon atoms to which they are attached, form a ring system it is preferred that, when $Ar^2$ is a naphthalene residue, the groups $R^6$ do not form a ring system. The groups $R^6$ are preferably alkyl groups containing from 1 to 24 carbon atoms, particularly from 1 to 6 carbon atoms or acyl groups containing from 2 to 24 carbon atoms, particularly from 2 to 6 carbon atoms as in acetyl, propanoyl and butanoyl groups. Thus, the group $Ar^2$ is preferably an unsubstituted naphthalene residue.

The infra-red absorbing phthalocyanine derivative may be a phthalocyanine or naphthalocyanine derivative. Compounds of this type are well known and are disclosed, inter alia, in European Patent Application No.155780 and Japanese Kokai 61 163891. The preferred phthalocyanine or naphthalocyanine derivatives are the metal phthalocyanine or naphthalocyanine derivatives, especially those in which the metal is copper since such derivatives are generally the most readily available and also are believed to be amongst the most stable of this type of product. However, other metal derivatives, or the dihydrogen derivatives, may be used.

The phthalocyanine or naphthalocyanine derivatives are typically of the general formula:

$(R^7 - D)_y - Pc$

wherein

Pc is a phthalocyanine or naphthalocyanine nucleus;

each $R^7$ is, independently, an optionally substituted aliphatic or aromatic radical linked to a peripheral carbon atom of the Pc nucleus through D;

D is selected from O, S, Se, Te, $NR^8$ and $PR^8$;

$R^8$ is a hydrogen atom or an alkyl group; and

y is from 4 to 16.

The group Pc may be metal-free or contain any of the metal or oxymetals which are capable of being complexed in the nucleus. Examples of suitable metals and oxymetals are magnesium, palladium, gallanyl,

vanadyl, germanium, indium and, more especially copper, nickel, cobalt, iron, zinc, lead and cadmium. Particularly preferred compounds are those in which the group Pc contains copper or nickel.

Each $R^7$ can be an aliphatic radical, for example an alkyl group containing 1 to 20 carbon atoms. However, it is preferred that each $R^7$ is preferably an optionally substituted mono- or bi-cyclic aryl or heteroaryl radical and is more preferably selected from phenyl, naphthyl, thienyl, furyl, pyrryl, thiazolyl, isothiazolyl, quinolyl, indolyl, pyridyl and benzothiazolyl which may carry one or more substitutents. Where $R^7$ is phenyl, the substituents may be situated in the ortho, meta and/or para positions with respect to the D linking atom. Any substituents which are present in the group $R^7$ are preferably selected from alkyl groups containing 1 to 20, especially 1 to 4, carbon atoms; alkoxy groups containing 1 to 20, especially 1 to 4, carbon atoms; thioalkyl groups containing 1 to 20, especially 1 to 4, carbon atoms; aryl; oxyaryl; thioaryl; halogen; nitro; cyano; tertiary amino such as dialkyl-, alkylaryl- and diaryl-amino, particularly those in which the alkyl group contains 1 to 4 carbon atoms and/or the aryl group is a phenyl group; an acyl such as one containing an alkyl group having 1 to 4 carbon atoms and a sulphonyl group such as one containing an alkyl group having 1 to 4 carbon atoms.

If the group D is $NR^8$ or $PR^8$, the group $R^8$ is preferably a hydrogen atom or an alkyl group of 1 to 20, particularly 1 to 4, carbon atoms and is especially hydrogen. However, the group D is preferably oxygen or, especially, sulphur.

It is preferred that the average value of y is from 6 to 16.

Other compounds which may be used with the organo-metallic compounds of the present invention are metal-aromatic amine complexes (hereinafter simply "metal amine"). The metal amine may be one of the general formula:

$$Ar^3X(NR^1)M(NR^1)XAr^3$$

wherein
M, $R^1$ and X are all as hereinbefore defined; and
$Ar^3$ is an aromatic hydrocarbon residue having at least two substituents;
where each X may be the same or different.

Metal amines are generally known and are disclosed, inter alia, in J.Amer.Chem.Soc., (1966), <u>88</u>, p 5201, and published Japanese Patent Application Kokai 61073902.

It is preferred that the metal M is a metal of Group VIII of the Periodic Table. It is especially preferred that M is selected from nickel, palladium or platinum. Useful materials have been obtained when M is nickel.

The group $Ar^3$ is typically of the type:

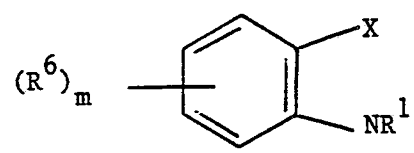

where:
$R^6$, X, $NR^1$ and m are all as hereinbefore defined.

Preferably the group X is a group $NR^1$ and it is especially preferred that the group X and the group $NR^1$ are the same.

The group $R^1$ may be a hydrocarbyl group such as an alkyl, aryl, alkylaryl, arylalkyl, alkenyl, cycloalkyl or cycloalkenyl group and typically contains from 1 to 24 carbon atoms, and particularly is an alkyl group containing up to six carbon atoms. However, it is generally preferred that the group $R^1$ is a hydrogen atom.

If the value of m is zero then, except for the groups X and $NR^1$, the group $Ar^3$ is an unsubstituted benzene ring. If two of the groups $R^6$, with the carbon atoms to which they are attached, form a ring system, this ring may be substituted with one or more substituents which are groups $R^6$. If the groups $R^6$ do not form a ring system they are preferably halogen atoms, alkyl groups containing from 1 to 24 carbon atoms, particularly from 1 to 6 carbon atoms, or a sulphonic acid group. Thus, the group $Ar^3$ may be, for example

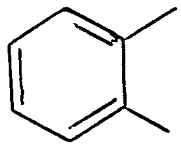
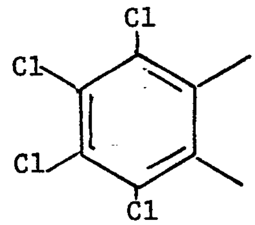
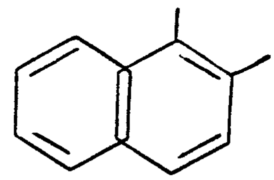

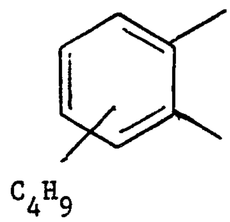

or

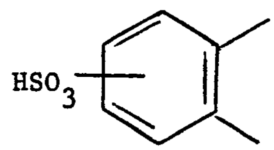

Alternatively, a metal dithiolethionedithiol complex, (hereafter simply "metal thione") may be included in a composition containing the organo-metallic compound of the present invention.

Metal thiones are generally known and compounds of this type are disclosed, inter alia, in Phosphorus and Sulphur (1979), 7, 49 and published Japanese Patent applications Kokai 61042585 and Kokai 61026686.

The metal thione is typically a compound of the formula

$[TS_2MS_2T]A$

where
A and M are both as hereinbefore defined; and
T is a residue which is derived from a dithiolethione dithiol.

Preferably the metal M and the cation A are as preferred in the organo-metallic compound of the present invention.

The group T may be derived from 1,2-dithiole-3-thione-4,5-dithiol or 1,3-dithiole-2-thione-4,5-dithiol.

Yet a further infra-red absorbing compound is an aminium or diimonium salt.

Aminium and diimonium salts are generally known and compounds of this type are disclosed, inter alia, in UK Patent Specification No.1069951 and United States Patent 3251881.

Aminium salts and diimonium salts are, respectively, of the general formulae I and II

$[(R^9)_2N - (Ph)_a - N^+(R^9)_2]An$ I

$[(R^9)_2N^+ = (Ph)_a = N^+(R^9)_2]2An$ II

where
An is a monovalent anion;
each $R^9$ which may be the same or different, is an amino-substituted aromatic group, which may be further substituted;
Ph is a para-phenylene group; and
a is one or two.

It is preferred that all of the groups $R^9$ are the same. It is preferred that each group $R^9$ is a para amino-substituted aromatic group. If the group $R^9$ is further substituted, the substituents may be halogen, hydroxyl, alkyl, alkoxy, alkylthio, amino and the like, where the alkyl group contains 1 to 30 carbon atoms. It is generally preferred that the group $R^9$ is not substituted. It is particularly preferred that each of the groups $R^9$ is a 4-aminophenyl group.

The anion An is a monovalent anion each molecule of the aminium salt contains one anion An and each molecule of the diimonium salt contains two anions An. The anion An may be a group $GF_6$, $ClO_4$, $R^{10}$-$SO_3$

and a halogen atom where
G is an element of group VB of the Periodic Table; and
$R^{10}$ is a hydrocarbyl group, which may be further substituted.

The group G is preferably arsenic or anitmony. $R^{10}$ may be substituted with groups such as halogen, acyl, $OR^{11}$, $SR^{11}$ or CN where $R^{11}$ is an alkyl group preferably containing 1 to 30 carbon atoms. The group $R^{10}$ may be an alkyl, aryl, alkylaryl, arylakyl, alkenyl, cycloalkyl or cycloalkenyl group. It is particularly preferred that the group $R^{10}$ is aryl or alkylaryl such as phenyl or 4-methylphenyl.

Some compositions containing the organo-metallic compounds of the present invention may result from the process used to obtain the organo-metallic compound. Other compositions can be obtained by mixing together at least one organo-metallic compound in accordance with the present invention with at least one further compound having infra-red absorbing properties, particularly the compounds discussed previously herein.

Any suitable mixing technique may be used. Thus, solid compounds may be ground together using a suitable grinding apparatus which may be for example a rotating ball mill or a vibrating ball mill. Alternatively, the compounds may be dissolved in suitable solvents such as, for example, a ketone, tetrahydrofuran, N,N-dimethyl- formamide, chloroform and the like, and the solutions obtained then mixed together.

The organo-metallic compounds of the present invention, or compositions including at least one such organo-metallic compound, may be incorporated into a polymeric material such as poly(vinyl butyral), polycarbonate, polyethyleneterephthalate (PET), polymethylmethacrylate, polyvinylchloride or polyethylene. Incorporation into the polymeric material is preferably effected by adding the infra-red absorbing compounds either as solids, or in solution, to the polymeric material. Solid mixing can be effected by grinding or using a high speed powder mixing device such as a Herschel mixer. If the infra-red absorbing materials are added as solutions to the polymeric material, the solutions are added to the solid material and the solvents are then evaporated. Alternatively, the infra-red absorbing materials may be incorporated into the polymeric material using a melt mixing technique, for example as in a Banbury mixer or an extruder.

Compositions containing the organo-metallic compounds of the invention can be obtained which absorb radiation of wavelength from 700 to about 2000 nm. Furthermore, such compositions generally have good transmission in the visible region of the spectrum. Hence such compositions are particularly suitable for applications which require good transmission in the visible region of the spectrum, together with absorption, preferably over a broad spectrum, in the infra-red region of the spectrum. The organo-metallic compounds of the present invention, or compositions which contain such organo-metallic compounds, can be used as infra-red absorbers in automobile or aircraft glass or in architectural glass either as a coating, for example in a suitable plastics material, on the glass or incorporated into the plastic interlayer, for example poly(vinyl butyral), in laminated glass. The compounds or compositions may be incorporated into plastic materials which have good light transmission properties such as polycarbonate, polyethyleneterephthalate (PET) or polymethylmethacrylate, for automobile or architectural use. Further uses are as an additive for greenhouse agricultural/horticultural film such as polyvinylchloride or polyethylene, in sunglasses or in solar energy collection devices, to absorb infra-red radiation from a laser or a semi-conductor light emitting diode, for example in printing or security inks for optical character recognition, for optical data storage or in optical filters on computer floppy discs, or in liquid crystal displays. Yet further uses are to aid in absorbing light from an infra-red heating lamp, for example to speed up bottle blowing using plastics materials such as polyethyleneterephthalate or in drying inks, paints or adhesives, or to provide protection against infra-red radiation in welding visors or goggles or to reduce the heat emitted from an incandescent lamp.

Many compounds which can be mixed with the organo-metallic compounds of the present invention are organic compounds or complexes with a transition metal, particularly with nickel. It has been found that the absorption characteristics of mixtures of the organo-metallic compounds of the present invention with such other compounds can change over a period of time, particularly if the mixture is dissolved in a suitable solvent such as methylene chloride or N,N-dimethylformamide and especially if the solution of the mixture is heated. This change has been attributed to a ligand exchange between the various transition metal complexes with the formation of a mixed complex which absorbs at a different wavelength.

A useful composition containing the organo-metallic compound of the present invention comprises pentadeca (4-methylbenzenethio) copper phthalocaynine, hexadeca (phenoxy) nickel phthalocyanine, the tetrabutylammonium salt of tetrachlorobenzenedithiol nickel complex, the tetrabutylammonium salt of 2-naphthol-1-thiol nickel complex and the di-tetrabutylammonium salt of bis(tetrachlorobenzenedithiol)(3,6-dichloro-1,2,4,5-tetrathiol)dinickel complex.

Preferred compositions are those in which the components have been mixed and allowed to equilibrate to allow any ligand exchange to occur and reach equilibrium. Thus, preferred compositions are those in

which the components have been mixed and maintained at a temperature of at least 100°C for at least one minute. The components are preferably maintained at the elevated temperature in a liquid phase which may be a material which is liquid at ambient temperature and also at the elevated temperature or a material which is solid at ambient temperature and molten at the elevated temperature, for example a polymeric material into which the composition of the present invention is being incorporated.

It is generally desirable that the components of the composition do not undergo appreciable ligand exchange. However, ligand exchange may be reduced by using separate layers, each layer containing at least one of the components of the composition or by using pigmentary absorbers.

Various aspects of the present invention are described in more detail in the following illustrative examples and by reference to Figures 1 to 9 which give the absorption spectra of compounds in accordance with the present invention at wavelengths of from 400 to 2000nm.

## Example 1

This example illustrates the preparation of 3,6-dichlorobenzene-1,2,4,5-tetrathiol by the process which is a further feature of the present invention.

A mixture of hexachlorobenzene (57g, 0.2 mol), sodium hydrosulphide (hydrated, available from Aldrich Chemical) (53.2g, 0.67 mol), sulphur (8g, 0.25 mol), iron powder (12g, 0.2 mol) and N,N-dimethylformamide (150 $cm^3$), was heated at 135°C with stirring, under nitrogen, for 10 hours. The mixture was cooled to about 90°C and water (500 $cm^3$) was added. A precipitate was formed which was filtered off and washed with water. The solid was then suspended in methanol (250 $cm^3$) with zinc oxide (20g), sodium hydroxide (50g) and water (250 $cm^3$). The mixture was heated at reflux with stirring for 1 hour, then cooled to room temperature and filtered. The filtrate was poured into a mixture of water (250 $cm^3$) and concentrated sulphuric acid (88 $cm^3$). A yellowish precipitate was formed which was filtered off, washed with water and ethanol, and then dried in vacuo (14mm of mercury) at 40°C. 16g of a solid having a melting point in the range 230-235°C were obtained.

## Example 2

The procedure of Example 1 was repeated with the exception that 54g of 1,2,4,5-tetrachlorobenzene was used to give a yield of 25g of benzene-1,2,4,5-tetrathiol.

## Example 3

3,6-dichlorobenzene-1,2,4,5-tetrathiol (5.5g, 0.02 mol) was dissolved in a mixture of N,N-dimethylformamide (100 $cm^3$) and tetrahydrofuran (100 $cm^3$). To this solution was added, with stirring, a solution of nickel (II) chloride hexahydrate (2.38g, 0.01 mol) in methanol (50 $cm^3$). Tetra-n-butylammonium bromide (7g, 0.022 mol) was then added and the mixture was stirred, without heating, for 3 hours whilst passing air through the mixture. The mixture was then concentrated by evaporation in vacuo (14mm) at 60°C to a volume of about 50 $cm^3$ and chloroform (300 $cm^3$) was added. A precipitate was formed which was filtered off, washed with chloroform and dried at 40°C and 14mm pressure. 2.5g of solid were obtained.

By analysis the composition was found to be C 42.8% wt; H 5.8% wt; N 2.0% wt; Cl 17.0% wt and S 23.5% wt. By calculation, the closest C, H, N and Cl values are achieved with a compound of the formula

$$[RS_2(NiS_2ArS_2)_{5.23}NiS_2R]\ 6.25Bu_4N$$

where

$S_2ArS_2$ is derived from 3,6-dichlorobenzene-1,2,4,5-tetrathiol,

$RS_2$ and $S_2R$ is derived from 3,4,5,6-tetrachlorobenzene-1,2-dithiol (present as an impurity in the tetrathiol; and

Bu is n-butyl,

the theoretical composition for this compound being C 44.7% wt; H 5.8% wt; N 2.2% wt; Cl 17.0% wt and S

20.8% wt.

0.2g of the solid were dissolved in N,N-dimethylformamide (1 $dm^3$). The absorption spectrum of the solution was determined at ambient temperature using a 1mm cell and a Perkin Elmer Lambda 9 spectrometer. The absorption spectrum, which shows a peak at 1950nm, is given in Figure 1.

Example 4

4,5-bis(benzoylthio)-1,3-dithiole-2-thione (0.81g) was added to 20 $cm^3$ of a 2M solution of sodium methoxide in methanol, the mixture was stirred for 10 minutes and was then added to a stirred solution of 3,6-dichlorobenzene-1,2,4,5-tetrathiol (0.28g) in a mixture of tetrahydrofuran (20 $cm^3$) and N,N-dimethylformamide (20 $cm^3$). A solution of nickel (II) chloride hexahydrate (0.48g) in methanol (10 $cm^3$) was then added, followed by tetra-n-butylammonium bromide (0.65g). The mixture was then stirred for 3 hours whilst passing air through the mixture. The mixture was then filtered to remove solid material and the filtrate was evaporated to dryness using a rotary evaporator under a water-pump vacuum at 60°C. The residue from the evaporation was suspended in water (100 $cm^3$), filtered, washed in turn with water and then ethanol and finally dried for 16 hours at 40°C and 14mm pressure. 0.5g of solid was obtained. From the U.V. and visible spectrum of the product, the following structure was attributed to the material obtained.

$[RS_2NiS_2ArS_2NiS_2R]\ 2Bu_4N$

where
$S_2ArS_2$ is derived from 3,6-dichlorobenzene-1,2,4,5-tetrathiol;
$RS_2$ and $S_2R$ is derived from 4,5-bis(benzoylthio)-1,3-dithiole-2-thione;
and
Bu is n-butyl.

The absorption spectrum was determined as described in Example 3 but using 0.1g of solid and a 1cm cell and the absorption spectrum is given in Figure 2.

Example 5

6-tert.butylphenol-2-thiol (2.73g, 0.015 mol), and 3,6-dichlorobenzene-1,2,4,5-tetrathiol (1.38g, 0.005 mol) were dissolved in a mixture of N,N-dimethylformamide (100 $cm^3$) and tetrahydrofuran (100 $cm^3$). A solution of nickel (II) chloride hexahydrate (2.38g, 0.01 mol) in methanol (50 $cm^3$) was added, followed by tetra-n-butylammonium bromide (3.22g, 0.01 mol). The mixture was stirred for 2 hours while air was blown through the solution. The solution was filtered. Ethanol (500 $cm^3$) was added to the filtrate, and a precipitate was formed which was collected by filtration and then washed with ethanol. The precipitate was then slurried in acetone (100 $cm^3$), filtered off, washed with acetone and dried at 14mm pressure and 40°C for 16 hours. A yield of 1g of solid product was obtained having an absorption spectrum, determined as set out in Example 4 but using 0.05g of solid, as shown in Figure 3. By analysis, and from the U.V. and visible spectrum of the product, the following structure was attributed to the material obtained.

$[RSONiS_2ArS_2NiOSR]\ 2Bu_4N$

where
$S_2ArS_2$ is derived from 3,6-dichlorobenzene-1,2,4,5-tetrathiol;
RSO and SRO is derived from 6-tert.butylphenol-2-thiol;
and
Bu is n-butyl.

Example 6

The procedure of Example 5 was repeated with the exception that the thiol and tetrathiol compounds were replaced by 3,4,5,6-tetrachlorobenzene-1,2-dithiol (2.8g, 0.010 mol) and benzene-1,2,4,5-tetrathiol (1.03g, 0.005 mol) respectively. After filtering the reaction mixture, the solution was evaporated to dryness and the solid residue was slurried in chloroform (100 $cm^3$). The solid was filtered off and washed well with

chloroform, then dried at 14mm pressure and 40°C for 16 hours. A yield of 1.1g of a solid product having a melting point in the range 229-231°C was obtained. The absorption spectrum, determined as described in Example 4 but using 0.025g of solid, is as shown in Figure 4, with an absorption maximum at 1235nm. The following structure was attributed to the material obtained.

$[RS_2NiS_2ArS_2NiS_2R]\ 2Bu_4N$

where
$S_2ArS_2$ is derived from benzene-1,2,4,5-tetrathiol;
$RS_2$ and $S_2R$ is derived from 3,4,5,6-tetrachlorobenzene-1,2-dithiol;
and
Bu is n-butyl.

## Example 7

The procedure of Example 5 was repeated with the exception that the 6-tert.butylphenol-2-thiol was replaced by quinoxaline-2,3-dithiol (1.94g, 0.015 mol). The spectrum of the compound showed an absorption peak at 660nm which suggested that oxidation of the nickel had not occurred.

The following structure was attributed to the material obtained.

$[RS_2NiS_2ArS_2NiS_2R]\ 4\ Bu_4N$

where
$S_2ArS_2$ is derived from 3,6-dichlorobenzene-1,2,4,5-tetrathiol;
$RS_2$ and $S_2R$ is derived from quinoxaline-2,3-dithiol;
and
Bu is n-butyl.

## Example 8

The Procedure of Example 5 was repeated using o-aminothiophenol (1.38g) to replace 6-tert.butylphenol-2-thiol. The absorption spectrum of the compound, determined as described in Example 5, is as shown in Figure 5, and shows an absorption peak at 1240nm.

The following structure was attributed to the material obtained.

$[R(NH)SNiS_2ArS_2NiS(NH)R]\ 2Bu_4N$

where
$S_2ArS_2$ is derived from 3,6-dichlorobenzene-1,2,4,5-tetrathiol;
R(NH)S and S(NH)R is derived from o-aminothiophenol; and
Bu is n-butyl.

## Example 9

3,4,5,6-tetrachlorobenzene-1,2-dithiol (16.0g) and 3,6-dichlorobenzene-1,2,4,5-tetrathiol (7.68g) were stirred in a mixture of tetrahydrofuran (400 $cm^3$) and N,N-dimethylformamide (400 $cm^3$). A solution of nickel (II) chloride hexahydrate (13.6g) in methanol (100 $cm^3$) was added to the stirred solution followed by a solution of sodium acetate (5.8g) in methanol (50 $cm^3$). The mixture was stirred for 3 hours while blowing air through the solution, then evaporated to dryness, slurried in ethanol (200 $cm^3$) and filtered. The solid was washed well with chloroform, then dried at 60°C and 14mm pressure for 16 hours. 10g of a solid having a melting point in the range 185 to 220°C was obtained having the following structure:

$[RS_2(NiS_2ArS_2)_xNiS_2R](x+1)\ Na$

where

$S_2ArS_2$ is derived from 3,6-dichlorobenzene-1,2,4,5-tetrathiol;
$RS_2$ and $S_2R$ is derived from 3,4,5,6-tetrachlorobenzene-1,2-dithiol;
and
x is zero or an integer.

The mixture was one in which the major portion was a compound in which the value of n is one, with a lesser proportion of a compound in which the value of n is two and small proportions in which the value of n is zero and is greater than two.

The absorption spectrum of the reaction product, determined as described in Example 4, using 0.02g of solid in 125 $cm^3$ of N,N-dimethylformamide, is as shown in Figure 6.

Similar mixtures can be obtained using the foregoing procedure and a mixture of di- and tetra-thiol derivatives obtained by the incomplete reaction of a polyhalobenzene using a process similar to that of Example 1 or Example 2.

Example 10

The procedure of Example 1 was repeated with the exception that the reaction temperature was 100°C, and the reaction mixture was hexafluorobenzene (18.6g), hydrated sodium hydrosulphide (32g), sulphur (4g), iron powder (6g) and N,n-dimethylformamide (100 $cm^3$).

13g of a product identified as 3,6-difluorobenzene-1,2,4,5-tetrathiol was obtained.

Example 11

The procedure of Example 10 was repeated on a reduced scale using hydrated sodium hydrosulphide (16g), sulphur (0.25g), iron powder (3g) and N,N-dimethylformamide (50 $cm^3$) and maintaining the temperature at 85°C for 1.5 hour.

3.85g of 3,4,5,6-tetrafluorobenzene-1,2-dithiol was obtained.

Example 12

The procedure of Example 5 was repeated with the exception that the thiol and tetrathiol compounds were replaced by 3,4,5,6-tetrachlorobenzene-1,2-dithiol (4.2g, 0.015 mol) and 3,6-difluorobenzene-1,2,4,5-tetrathiol (1.21g, 0.005 mol) respectively. The reaction mixture, without being filtered, was evaporated to dryness, the solid residue was suspended in ethanol (50 $cm^3$) and filtered. The solid was washed in turn with ethanol and then with chloroform, which treatment removed essentially all of the bis-(tetrachlorobenzene-1,2-dithiol) nickel complex. The solid product was dried at 14mm pressure and 40°C for 12 hours. A yield of 1.9g of a solid was obtained to which the following structure was attributed

$[RS_2NiS_2ArS_2NiS_2R]\ 2Bu_4N$

where
$S_2ArS_2$ is derived from 3,6-difluorobenzene-1,2,4,5-tetrathiol;
$RS_2$ and $S_2R$ is derived from 3,4,5,6-tetrachlorobenzene-1,2-dithiol;
and
Bu is n-butyl.

The absorption spectrum, which was determined as described in Example 6, is as shown in Figure 7.

Example 13

Hexachlorobenzene (5.7g, 200 mmol), hydrated sodium hydrosulphide (6.4g, about 80 mmol), sulphur (0.8g), iron powder (1.2g) and N,N-dimethylformamide (50 $cm^3$) were stirred together under nitrogen at 135°C for ten hours. Nickel chloride hexahydrate (4.76g, 20 mmol) and tetra-n-butylammonium bromide (6.44g, 20 mmol) were then added and the mixture was stirred for a further 30 minutes at 135°C. A further quantity of N,N-dimethylformamide (50 $cm^3$) was added to the mixture, the hot solution was filtered and the filtrate cooled to ambient temperature. Ethanol (100 $cm^3$) was added and a precipitate was formed which

was filtered off, washed with ethanol and chloroform and dried at 40°C and 14mm pressure for 16 hours. 4.5g of a solid product were obtained having an electronic absorption spectrum (0.01% w/v in N,N-dimethylformamide, and 1cm cell) as shown in Figure 8.

The following structure was attributed to the material obtained.

$[RS_2(NiS_2ArS_2)_xNiS_2R]$ (x+1) $Bu_4N$

where
$S_2ArS_2$ is derived from 3,6-dichlorobenzene-1,2,4,5-tetrathiol;
$RS_2$ and $S_2R$ is derived from 3,4,5,6-tetrachlorobenzene-1,2-dithiol;
Bu is n-butyl; and
x is zero or an integer.
The mixture was one in which the major portion was a compound in which the value of n is one and small proportions of compound in which the value of n is zero or is greater than one.

Example 14

The procedure of Example 3 was repeated with the exception that the 3,6-dichlorobenzene-1,2,4,5-tetrathiol was replaced by an equal molar quantity of benzene-1,2,4,5-tetrathiol.

0.573g of the product thus obtained and 1.285g of tetra-n-butylammonium bis(tetrachlorobenzene-1,2-dithiolato) nickel (III) were refluxed in 20 $cm^3$ of N,N-dimethylformamide for one hour. The N,N-dimethylformamide was evaporated from the mixture at water-pump pressure and a temperature of about 60°C. The residue was washed well with chloroform and dried at 40°C and 1mm pressure for 2 hours.

The product had an electron absorption spectrum (0.0025% w/v in N,N-dimethylformamide using a one cm cell) as shown in Figure 9.

Example 15

3,4,5,6-tetrachlorobenzene-1,2-dithiol (2.8g, 0.01 mol), and 3,6-dichlorobenzene-1,2,4,5-tetrathiol (1.38g, 0.005 mol) were dissolved in a mixture of N,N-dimethylformamide (75 $cm^3$) and tetrahydrofuran (75 $cm^3$). A solution of nickel (II) chloride hexahydrate (2.38g, 0.01 mol) in methanol (20 $cm^3$) was added, followed by tetra-n-butylammonium bromide (3.22g, 0.01 mol). The mixture was stirred for 2 hours while air was blown through the solution. The solution was filtered. Meths was added to the filtrate, and a precipitate was formed which was collected by filtration and then washed with chloroform and then meths. The precipitate was dried at 14mm pressure and 40°C for 16 hours. A yield of 1.3g of solid product was obtained. The following structure was attributed to the material obtained.

$[RS_2NiS_2ArS_2NiS_2R]$ $2Bu_4N$

where
$S_2ArS_2$ is derived form 3,6-dichlorobenzene-1,2,4,5-tetrathiol;
$RS_2$ and $S_2R$ is derived from 3,4,5,6-tetrachlorobenzene-1,2-dithiol;
and
Bu is n-butyl.

Examples16 and 17 which follow illustrate compositions containing an organo-metallic compound in accordance with the present invention together with other infra-red absorbing materials. Figures 10 to 13 give absorption and transmission spectra for the compositions at wavelengths of from 300 to1600 nm.

Example 16

A mixture was prepared by grinding together, using a mortar and pestle, the product of Example 15 (15), pentadeca(4-methylbenzenethio) copper phthalocyanine (A), the tetrabutylammonium salt of tetrachlorobenzenedithiol nickel complex (B) and the tetrabutylammonium salt of 2-naphthol-1-thiol nickel complex (C). The solids were ground together in amounts to give 1 mole of (15), 0.25 mole of (A), 8 mole of (B) and 2 mole of (C). Grinding was continued to obtain the mixed solids as a fine powder.

100mg of the powdered mixture was dissolved in one $dm^3$ of N,N-dimethylformamide. The mixture was heated to reflux temperature for one minute and then allowed to cool. The absorption spectrum of the resulting solution is given in Figure 10. The transmission characteristics of the mixture are given in Figure 11.

Example 17

The procedure of Example 16 was repeated using a total of five components to obtain a mixture of 7.0% molar of (15), 1.5% molar of (A), 3.6% molar of hexadeca (phenoxy) nickel phthalocyanine, 71.7% molar of (B) and 16.1% molar of (C).

The absorption spectrum of the mixture was determined as described in Example 16. The absorption spectrum is given in Figure 12. The percentage transmission spectrum was determined and is given in Figure 13.

## Claims

1. An organo-metal complex which is at least one compound of the formula:

$$[RYZ(MXYArXY)_xMYZR]\ A$$

wherein:

A is a cation having a valency, and being in an amount, to give a neutral compound;
Ar is an aromatic hydrocarbon or heterocyclic residue having at least four substituents;
R is a hydrocarbon residue having at least two substituents;
M is a transition metal;
X is a sulphur atom or a group $NR^1$;
Y is a sulphur atom, an oxygen atom or a group $NR^1$;
Z is a sulphur atom, an oxygen atom or a group $NR^1$;
$R^1$ is a hydrogen atom or a hydrocarbyl group; and
x is an integer having a value of from 1 up to 10,
and X, Y and Z may be the same or different.

2. A complex as claimed in claim 1 wherein A is a monovalent cation selected from an ammonium group or an alkali metal and each molecule of the complex contains (x+1) molecules of the cation A.

3. A complex as claimed in either claim 1 or claim 2 wherein Ar contains further substituents which are different from X and Y and which are halogen, alkyl, acyl, aryl, $-SR^2$ or CN where $R^2$ is an alkyl group.

4. A complex as claimed in any one of claims 1 to 3 wherein M is nickel.

5. A complex as claimed in any one of claims 1 to 4 wherein R is of the general formula:

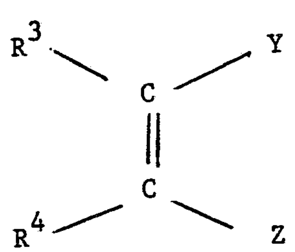

wherein:
$R^3$ and $R^4$ may be the same or different and are hydrogen, hydrocarbyl, substituted hydrocarbyl, or heterocyclic or $R^3$ and $R^4$, together with the two carbon atoms to which they are attached, may form a ring system.

6. A complex as claimed in any one of claims 1 to 5 wherein X, Y and Z are all sulphur atoms.

7. A mixture of compounds which are organo-metal complexes as claimed in any one of claims 1 to 6 and in which the value of x is different.

8. A composition which comprises a complex as claimed in any one of claims 1 to 6 or a mixture as claimed in claim 7 and at least one material having infra-red absorbing characteristics and selected from compounds of the formula:

[RYZMYZR]A ,

phthalocyanine derivatives, metal-aromatic amine complexes, metal dithiolethionedithiol complexes, and aminium or diimonium salts.

9. A process for preparing an organo-metallic compound which comprises reacting a salt of a metal M with a compound of the formula:

$(HY)_2Ar(XH)_2$

and a compound of the formula:

HYRZH

wherein:
Ar is an aromatic hydrocarbon or heterocyclic residue having at least four substituents;
R is a hydrocarbon residue having at least two substituents;
M is a transition metal;
X is a sulphur atom or a group $NR^1$;
Y is a sulphur atom, an oxygen atom or a group $NR^1$;
Z is a sulphur atom, an oxygen atom or a group $NR^1$;
$R^1$ is a hydrogen atom or a hydrocarbyl group; and
X, Y and Z may be the same or different.

10. A process as claimed in claim 9 wherein the compound of the formula:
$(HY)_2Ar(XH)_2$

is
benzene-1,2,4,5-tetrathiol;
3,6-dichlorobenzene-1,2,4,5-tetrathiol;
naphthalene-1,2,5,6-tetrathiol;
pyridine-2,3,5,6-tetrathiol;
3,6-difluorobenzene-1,2,4,5-tetrathiol;
1,4-dihydroxybenzene-2,5-dithiol;
1,3-dihydroxybenzene-4,6-dithiol;
1,2,4,5-tetraaminobenzene;
1,3-diaminobenzene-4,6-dithiol;
1,4-diaminobenzene-2,6-dithiol; or
2,6-dihydroxynaphthalene-1,5-dithiol.

11. A process as claimed in either claim 9 or claim 10 wherein the compound of the formula HYRZH is
3,4,5,6-tetrachlorobenzene-1,2-dithiol;
naphthalene-1,2-dithiol;
2-hydroxynaphthalene-1-thiol;
aniline-2-thiol;
4-butyl-1,2-diaminobenzene;
quinoxaline-2,3-dithiol;
1,2-diphenylethylene-1,2-dithiol(cis);
1,3-dithiole-2-thione-4,5-dithiol; or
6-tert.butylphenol-2-thiol.

12. A process as claimed in any one of claims 9 to 11 which includes an oxidation step.

13. A process for preparing a tetrathiol compound which comprises reacting an aromatic compound containing at least four halogen substituents attached to the aromatic ring with a metal hydrosulphide and sulphur in the presence of iron powder or an iron salt in a polar organic solvent wherein for each mole of the aromatic compound there are used at least 4 moles of the metal hydrosulphide, at least 0.5 moles of sulphur and at least 0.5 moles of iron powder or iron salt.

14. An infra-red absorbing composition which contains at least one organo-metal complex as claimed in any one of claims 1 to 6 or a mixture as claimed in either claim 7 or claim 8.

15. A plastics material containing an infra-red absorbing composition as claimed in claim 14.

Claims for the following Contracting State: ES

1. A process for preparing an organo-metallic compound which comprises reacting a salt of a metal M with a compound of the formula:

$(HY)_2Ar(XH)_2$

and a compound of the formula:

HYRZH

wherein:

Ar is an aromatic hydrocarbon or heterocyclic residue having at least four substituents;
R is a hydrocarbon residue having at least two substituents;
M is a transition metal;
X is a sulphur atom or a group $NR^1$;
Y is a sulphur atom, an oxygen atom or a group $NR^1$;
Z is a sulphur atom, an oxygen atom or a group $NR^1$;
$R^1$ is a hydrogen atom or a hydrocarbyl group; and
X, Y and Z may be the same or different.

2. A process as claimed in claim 1 wherein the compound of the formula:

$(HY)_2Ar(XH)_2$

is
benzene-1,2,4,5-tetrathiol;
3,6-dichlorobenzene-1,2,4,5-tetrathiol;
naphthalene-1,2,5,6-tetrathiol;
pyridine-2,3,5,6-tetrathiol;
3,6-difluorobenzene-1,2,4,5-tetrathiol;
1,4-dihydroxybenzene-2,5-dithiol;
1,3-dihydroxybenzene-4,6-dithiol;
1,2,4,5-tetraaminobenzene;
1,3-diaminobenzene-4,6-dithiol;
1,4-diaminobenzene-2,5-dithiol; or
2,6-dihydroxynaphthalene-1,5-dithiol.

3. A process as claimed in either claim 1 or claim 2 wherein the compound of the formula HYRZH is
3,4,5,6-tetrachlorobenzene-1,2-dithiol;
naphthalene-1,2-dithiol;
2-hydroxynaphthalene-1-thiol;
aniline-2-thiol;
4-butyl-1,2-diaminobenzene;
quinoxaline-2,3-dithiol;
1,2-diphenylethylene-1,2-dithiol(cis);
1,3-dithiole-2-thione-4,5-dithiol; or
6-tert.butylphenol-2-thiol.

4. A process as claimed in any one of claims 1 to 3 wherein the salt of a metal M is nickel chloride.

5. A process as claimed in any one of claims 1 to 4 which include an oxidation step.

6. A process as claimed in any one of claims 1 to 5 wherein the molar proportions of the salt of the metal M, the compound $(HY_2Ar(XH)_2$ and the compound HYRZH are about 2:1:2 to 3:2:2.

7. A process as claimed in any one of claims 1 to 6 wherein a mixture of compounds is obtained.

8. A process for preparing a tetrathiol compound which comprises reacting an aromatic compound containing at least four halogen substituents attached to the aromatic ring with a metal hydrosulphide and sulphur in the presence of iron powder or an iron salt in a polar organic solvent wherein for each mole of the aromatic compound there are used at least 4 moles of the metal hydrosulphide, at least 0.5 moles of sulphur and at least 0.5 moles of iron powder or iron salt.

9. A process as claimed in claim 8 wherein an iron complex is formed initially and this is then reacted with a nickel compound in the presence of a salt or base.

10. A composition which comprises at least two different compounds each of which is of the formula:

$$[RYZ(MXYArXY)_xMYZR]\ A$$

wherein:

A is a cation having a valency, and being in an amount, to give a neutral compound;
Ar is an aromatic hydrocarbon or heterocyclic residue having at least four substituents;
R is a hydrocarbon residue having at least two substituents;
M is a transition metal;
X is a sulphur atom or a group $NR^1$;
Y is a sulphur atom, an oxygen atom or a group $NR^1$;
Z is a sulphur atom, an oxygen atom or a group $NR^1$;
$R^1$ is a hydrogen atom or a hydrocarbyl group; and
x is an integer having a value of from 1 up to 10; and
X, Y and Z may be the same or different;
wherein at least one of A, Ar, R, M, X, Y, Z, $R^1$ and x is different in each compound.

11. A composition as claimed in claim 10 wherein M is nickel in each of the compounds.

12. A composition as claimed in either claim 10 or claim 11 wherein X, Y and Z are all sulphur atoms in each of the compounds.

13. A composition as claimed in any one of claims 10 to 12 in which the value of x is different in each of the compounds.

14. A composition which comprises at least one compound of the formula:

$$[RYZ(MXYArXY)_xMYZR]\ A$$

wherein:

A is a cation having a valency, and being in an amount, to give a neutral compound;
Ar is an aromatic hydrocarbon or heterocyclic residue having at least four substituents;
R is a hydrocarbon residue having at least two substituents;
M is a transition metal;
X is a sulphur atom or a group $NR^1$;
Y is a sulphur atom, an oxygen atom or a group $NR^1$;
Z is a sulphur atom, an oxygen atom or a group $NR^1$;
$R^1$ is a hydrogen atom or a hydrocarbyl group;
x is an integer having a value of from 1 up to 10; and
X, Y and Z may be the same or different;
and at least one further material which is a compound having infra-red absorbing characteristics and is selected from compounds of the formula:

$$[RYZMYZR]A\ ,$$

phthalocyanine derivatives, metal-aromatic amine complexes, metal dithiolethionedithiol complexes, and aminium or diimonium salts.

15. A composition which is a plastics material, a printing or security ink, an adhesive or a paint and which contains at least one organo-metallic compound of the formula:

$$[RYZ(MXYArXY)_xMYZR]\ A$$

wherein:

A is a cation having a valency, and being in an amount, to give a neutral compound;
Ar is an aromatic hydrocarbon or heterocyclic residue having at least four substituents;
R is a hydrocarbon residue having at least two substituents;

M is a transition metal;
X is a sulphur atom or a group $NR^1$;
Y is a sulphur atom, an oxygen atom or a group $NR^1$;
Z is a sulphur atom, an oxygen atom or a group $NR^1$;
$R^1$ is a hydrogen atom or a hydrocarbyl group;
x is an integer having a value of from 1 up to 10; and
X, Y and Z may be the same or different;
or a composition as claimed in claim 14.

Fig.1.

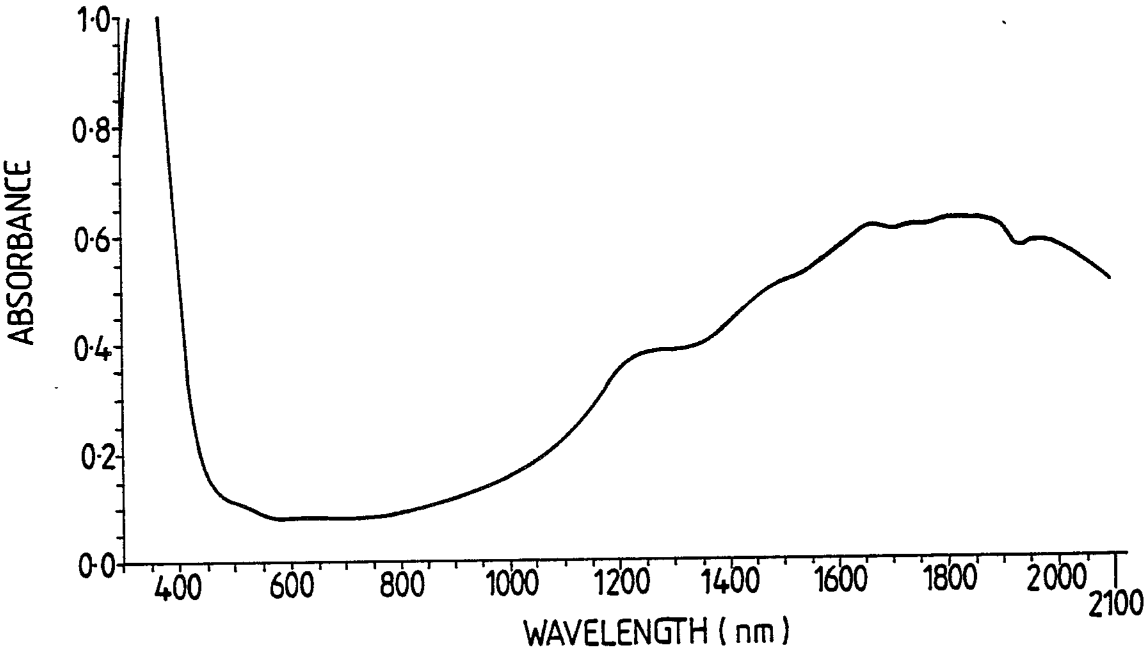
1·0
0·8
0·6
0·4
0·2
0·0
ABSORBANCE
400
600
800
1000
1200
1400
1600
1800
2000
2100
WAVELENGTH ( nm )

Fig.2.

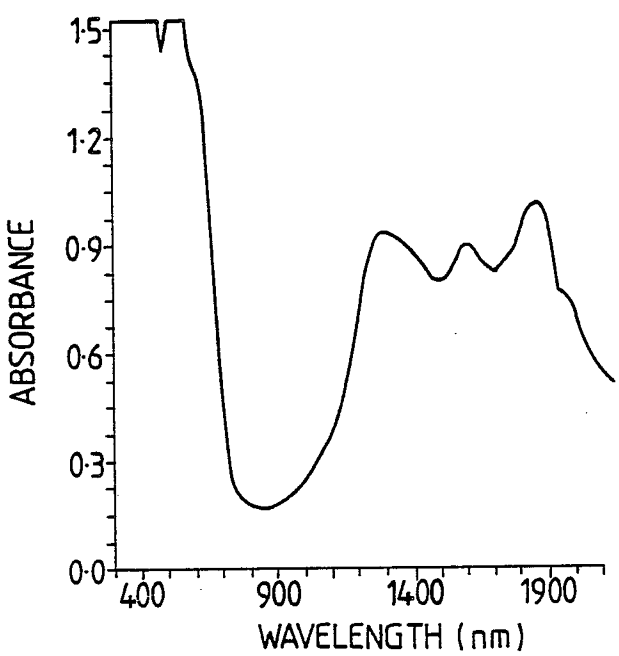
1·5
1·2
0·9
0·6
0·3
0·0
ABSORBANCE
400
900
1400
1900
WAVELENGTH ( nm )

Fig. 3.

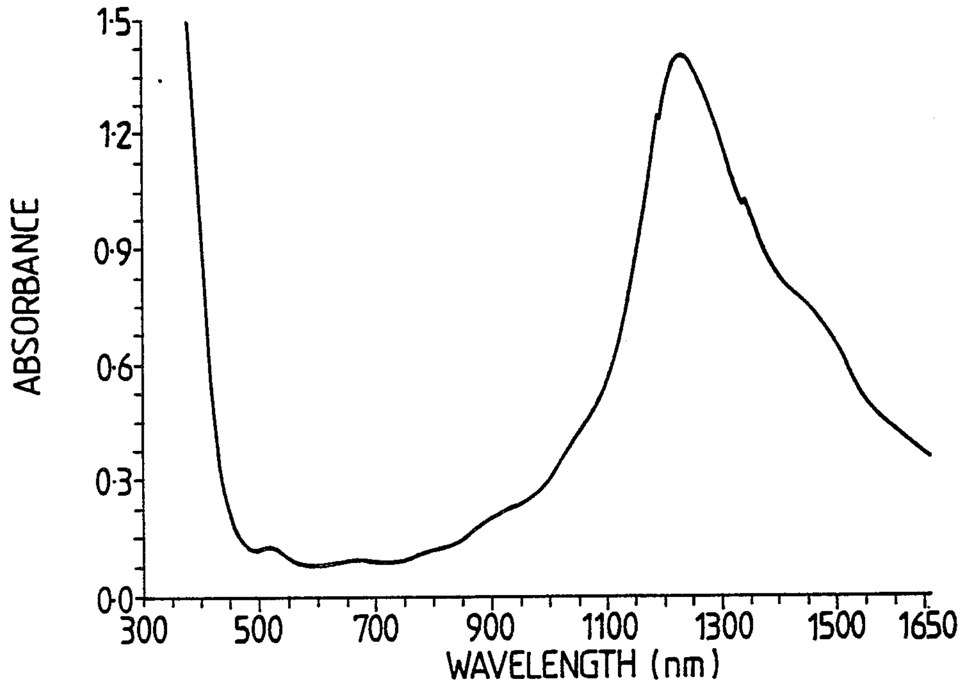
1·5
1·2
0·9
0·6
0·3
0·0
ABSORBANCE
300
500
700
900
1100
1300
1500
1650
WAVELENGTH (nm)

Fig. 4.

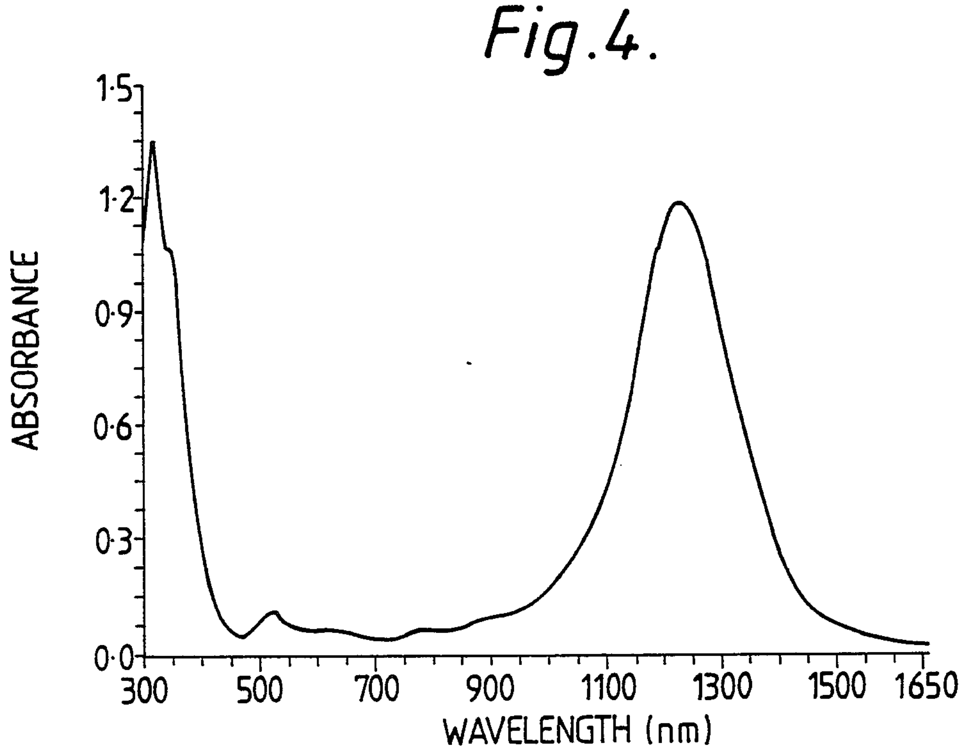
1·5
1·2
0·9
0·6
0·3
0·0
ABSORBANCE
300
500
700
900
1100
1300
1500
1650
WAVELENGTH (nm)

Fig. 5.

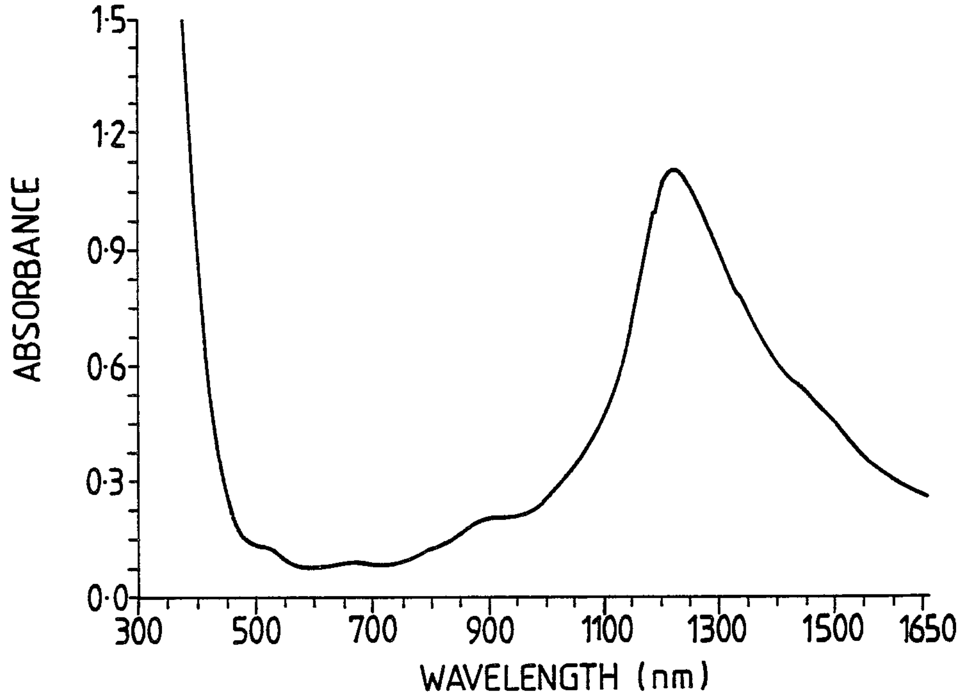
ABSORBANCE
1·5
1·2
0·9
0·6
0·3
0·0
300
500
700
900
1100
1300
1500
1650
WAVELENGTH (nm)

Fig. 6.

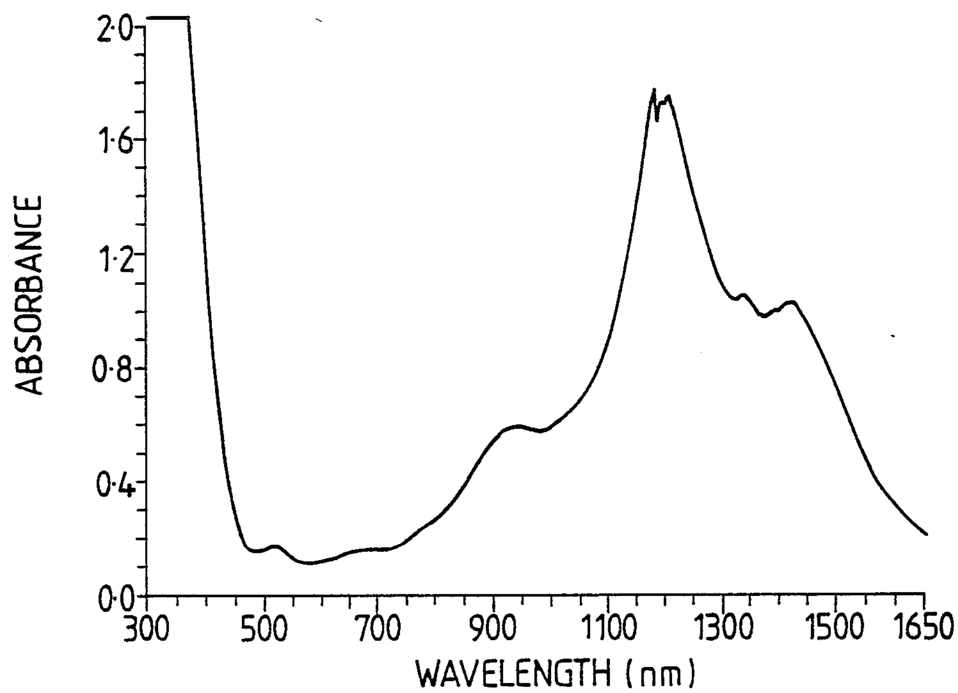
ABSORBANCE
2·0
1·6
1·2
0·8
0·4
0·0
300
500
700
900
1100
1300
1500
1650
WAVELENGTH (nm)

Fig. 7.

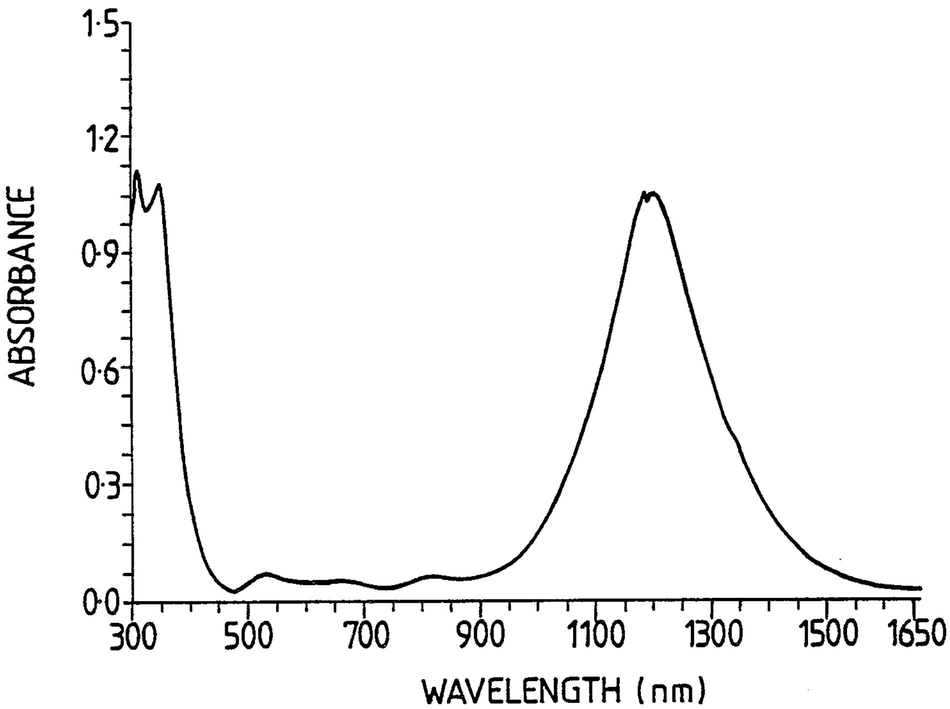
1·5
1·2
0·9
0·6
0·3
0·0
ABSORBANCE
300
500
700
900
1100
1300
1500
1650
WAVELENGTH (nm)

Fig. 8.

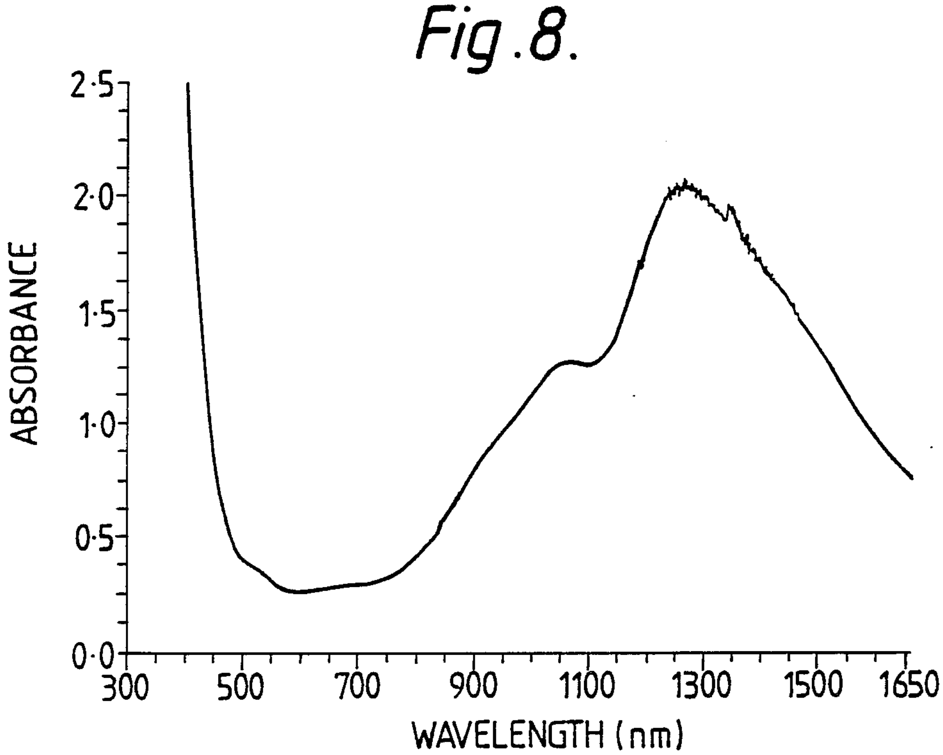
2·5
2·0
1·5
1·0
0·5
0·0
ABSORBANCE
300
500
700
900
1100
1300
1500
1650
WAVELENGTH (nm)

Fig. 9.

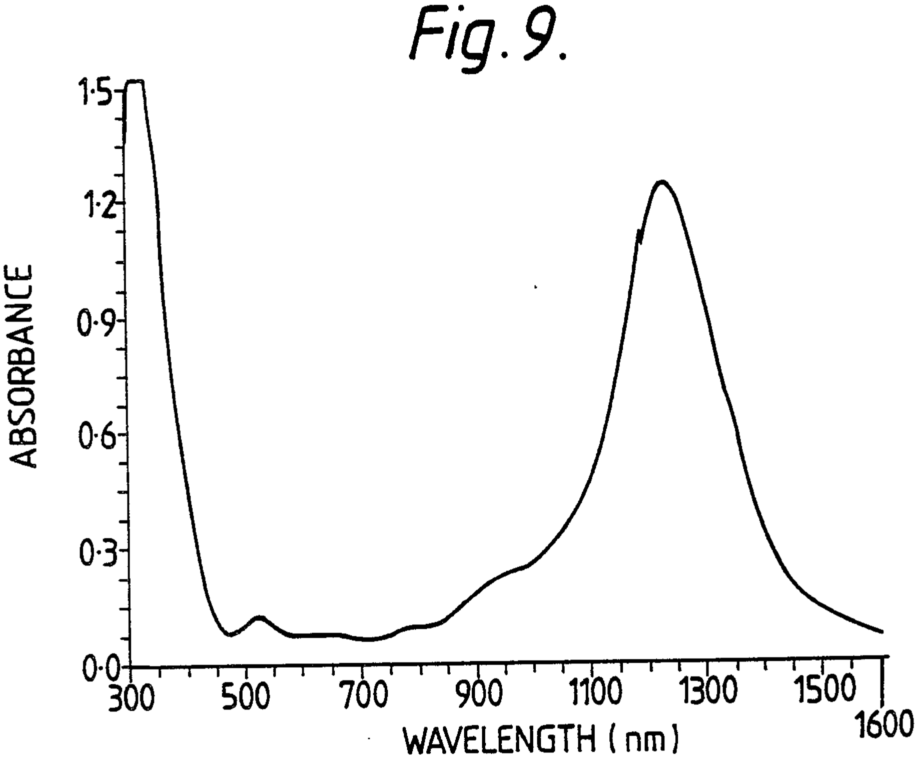


Fig. 11.

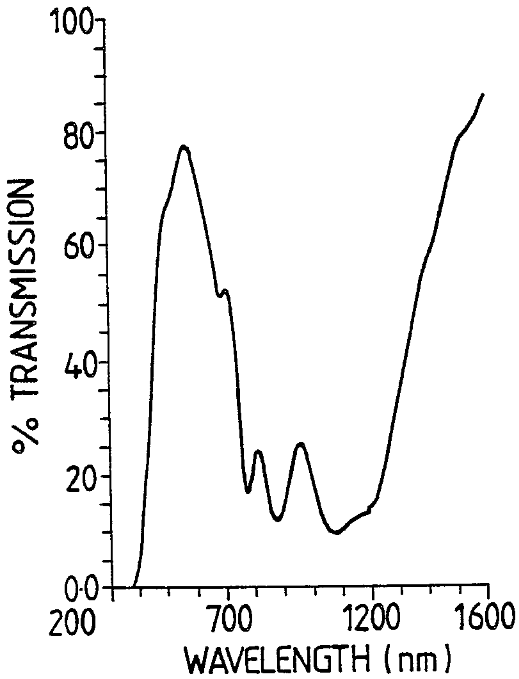


Fig. 10.

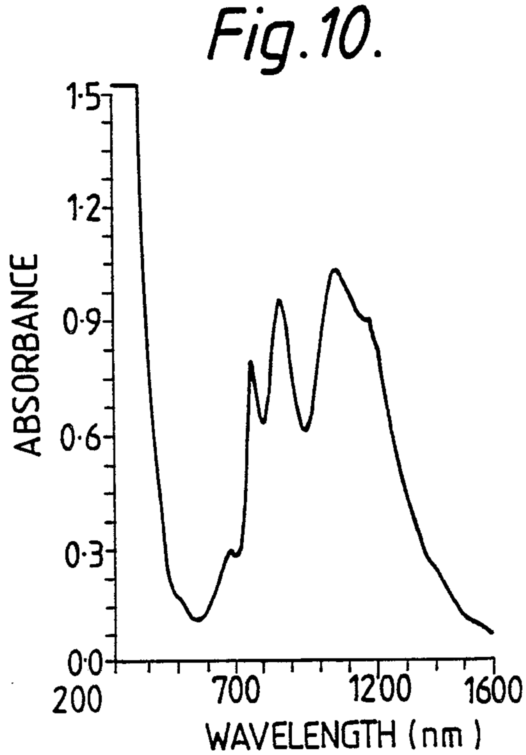

Fig.13.

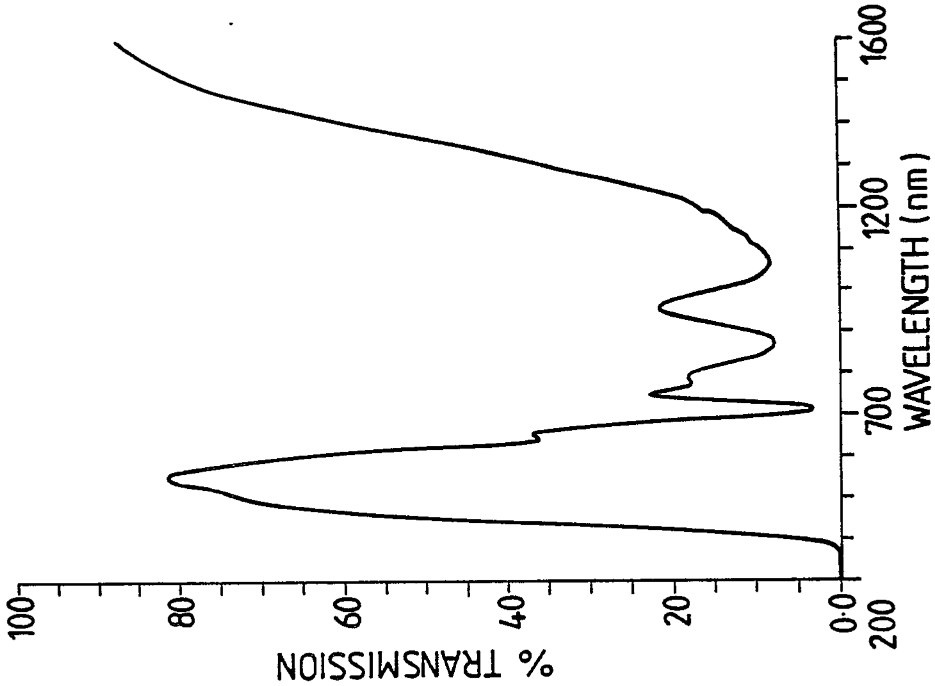

100
80
60
40
20
0·0
% TRANSMISSION
200
700
1200
1600
WAVELENGTH (nm)


Fig.12.

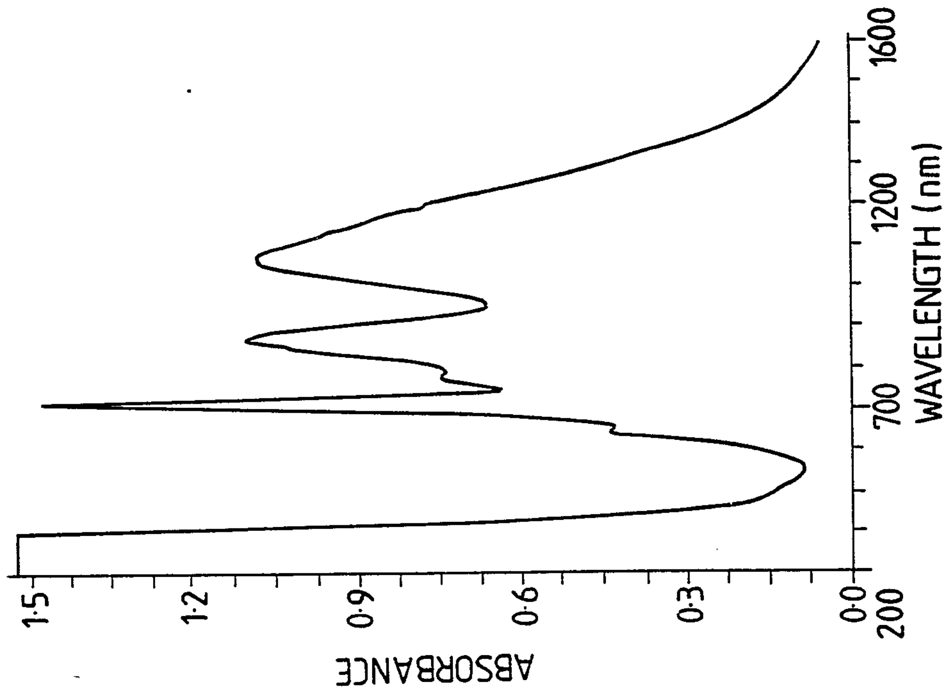

1·5
1·2
0·9
0·6
0·3
0·0
ABSORBANCE
200
700
1200
1600
WAVELENGTH (nm)